# EUROPÄISCHE PATENTSCHRIFT

(11) **EP 0 971 931 B1**
(45) Veröffentlichungstag und Bekanntmachung des Hinweises auf die Patenterteilung: **19.02.2003**
(21) Anmeldenummer: 98916981.8
(22) Anmeldetag: 17.03.1998
(51) Int. Cl.: C07D 495/04

(54) **VERFAHREN ZUR ENTBENZYLIERUNG VON DIBENZYLBIOTIN**
METHOD FOR DEBENZYLATION OF DIBENZYL BIOTIN
PROCEDE DE DEBENZYLATION DE BIOTINE DE DIBENZYLE

(30) Priorität: 27.03.1997 DE 19712952
(43) Veröffentlichungstag der Anmeldung: 19.01.2000
(73) Patentinhaber: MERCK PATENT GmbH, 64271 Darmstadt (DE)
(72) Erfinder: HEYWANG, Ulrich, D-64289 Darmstadt (DE); BOLLINGER, Heinrich, CH-8222 Beringen (CH); MÜLLER, Hans-Rudolf, CH-8207 Schaffhausen (CH)
(86) Internationale Anmeldenummer: EP9801545
(87) Internationale Veröffentlichungsnummer: WO98043979

(56) Entgegenhaltungen:
- EP-A- 0 564 723
- US-A- 4 537 973

## Beschreibung

Die vorliegende Erfindung betrifft ein neues Verfahren zur selektiveren Entbenzylierung von Dibenzylbiotin, das als Zwischenprodukt in der Biotinsynthese gebildet wird, üblicherweise jedoch nicht isoliert wird.

D-(+)-Biotin wird in einem vielstufigen Verfahren, zumeist nach Varianten von Gerecke hergestellt, wie von Gerecke, Zimmermann und Aschwanden in Heiv Chim. Acta **53** (1970) 991 ff beschrieben. All diesen Methoden ist gemeinsam, daß am Ende der Synthese Dibenzylbiotin (Formel (I) entsteht, welches jedoch oft nicht isoliert wird.

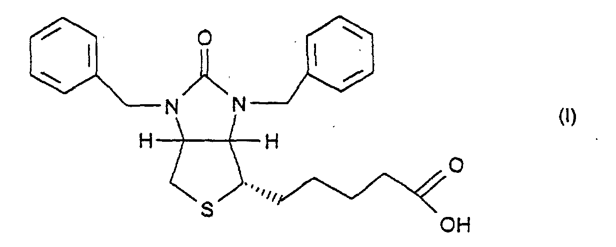

Die Entfernung der Benzylgruppen ist bisher nur in einem aufwendigen Prozeß von mehreren Verfahrensschritten möglich. Aus US-A-4537973 sind Debenzylierungsreaktionen unter Einsatz von Methansulfonsäure bekannt, welche jedoch nur im Labormaßstab einsetzbar sind. Entsprechende Umsetzungen mit Natriumamalgam sind aus Umweltschutzgründen nicht mehr zulässig. Fast ausschließlich erfolgt daher diese Umsetzung, insbesondere im großtechnischen Maßstab, mit Bromwasserstoffsäure, in Ausnahmefällen auch mit lodwasserstoffsäure. Letztere ist jedoch aus Kostengründen nicht im großtechnischen Maßstab durchführbar.

EP-A-0564723 beschreibt die Entbenzylierung von Dibenzylbiotin mit Bromwasserstoffsäure.

Umsetzungen mit Bromwasserstoffsäure führen zwar zu Ausbeuten von etwa 90 % der Theorie, weisen jedoch gravierende Nachteile auf. Hierzu zählen neben dem hohen Preis der Bromwasserstoffsäure, lange Reaktionszeiten von 24 bis 36 Stunden, wobei die Temperatur bei 145 °C gehalten werden muß, auch die Bildung von unreinem, tränenreizendem Benzylbromid welches entweder zur weiteren

Verwendung aufgereinigt oder mit Natronlauge entgiftet werden muß. außerdem erfolgt als Nebenreaktion eine Decarbonylierung des Biotinmoleküls, wodurch etwa 50 % des Reaktionsprodukts verloren gehen und durch einen weiteren Verfahrensschritt in einer Umsetzung mit Phosgen wieder in das Molekül eingeführt werden muß:

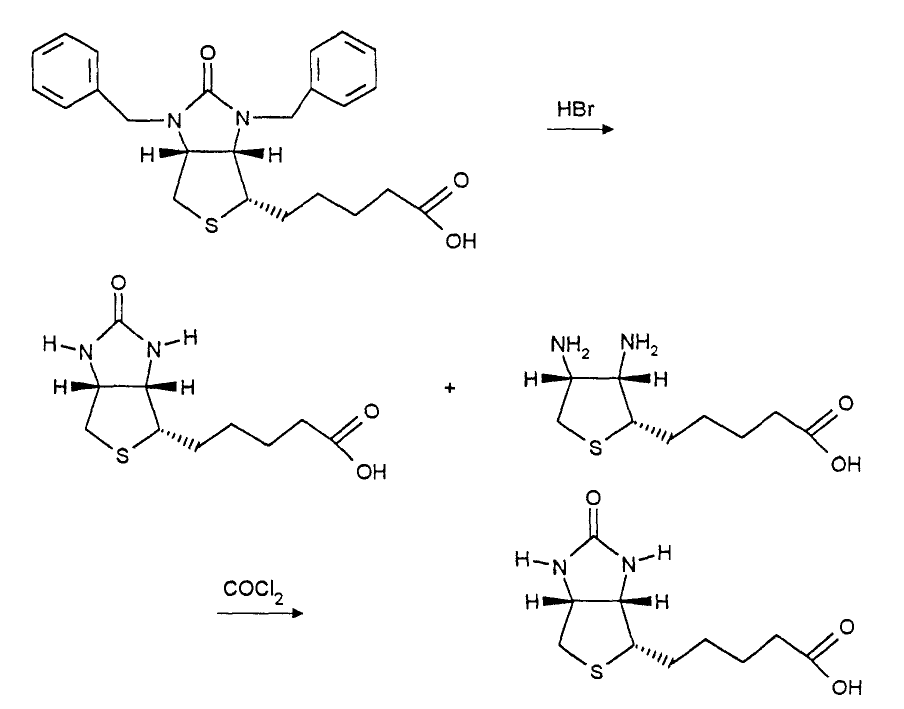

Nach Durchführung dieser Verfahrensschritte wird aus dem mit einer Reinheit von 85 % eingesetzten Dibenzylbiotin Biotin mit einer Reinheit von etwa 93 % in einer Ausbeute von 90 % der Theorie erhalten. Bei den im Produkt enthaltenen Verunreinigungen handelt es sich um eine Vielzahl von Verbindungen, weiche durch ungeklärte Nebenreaktionen entstanden sind.

Aufgabe der vorliegenden Erfindung ist es daher, ein in einfacher Weise , bei niedrigeren Temperaturen und verkürzter Reaktionsdauer durchführbares Verfahren zur Entbenzylierung von Dibenzylbiotin (Formel (I) zur Verfügung zu stellen, welches unter Verwendung von preiswerten Reagenzien abläuft und mit hohen Ausbeuten zu D-(+)-Biotin mit hoher Reinheit führt.

Die Lösung der Aufgabe erfolgt durch ein Verfahren zur Herstellung von D-(+)-Biotin durch selektive Abspaltung von Benzylgruppen, indem
a) eine wäßrige Lösung des als Zwischenprodukt gebildeten Dibenzylbiotins mit Schwefelsäure versetzt wird,
b) unerwünschte Neben- und Spaltprodukte nach Einstellung eines neutralen bis alkalischen pH-Werts mit Hilfe eines organischen Lösungsmittels extrahiert werden und
c) das freigesetzte D-(+)-Biotin durch Einstellung eines sauren pH-Werts und Erniedrigung der Temperatur auskristallisiert und abgetrennt wird.

In diesem Verfahren kann vorteilhafterweise erfindungsgemäß ungereinigtes Dibenzylbiotin verwendet werden, welches beispielsweise als Zwischenprodukt in dem durch DE-A1-4411101 beschriebenen Verfahren erhalten wird. Der nach Abtrennung des Hydrierkatalysators und Abdestillieren des Lösungsmittels erhaltene Rückstand kann direkt in dem hier beschriebenen erfindungsgemäßen Verfahren eingesetzt werden.
Schwefelsäure, wird insbesondere als 70 bis 80 %ige Schwefelsäure verwendet.
Das erfindungsgemäße Verfahren ist dadurch gekennzeichnet, daß die Zugabe der Schwefelsäure bei einer Temperatur von 25 bis 115 °C erfolgt.
Durchgeführt werden kann dieses Verfahren, indem nach beendeter Reaktion unerwünschte Neben- und Spaltprodukte nach Einstellung eines neutralen bis alkalischen pH-Werts mit Hilfe eines geeigneten organischen Lösungsmittels aus der Gruppe Toluol, Xylol und extrahiert werden und nach erfolgter Lösungsmittelextraktion der pH-Wert schwach sauer, insbesondere auf einen pH-Wert von 6, eingestellt wird, und die wäßrige D-(+)-Biotin-haltige Lösung mit Aktivkohle behandelt wird. Das freigesetzte D-(+)-Biotin kann anschließend nach dem erfindungsgemäßen Verfahren nach der Einstellung des pHs auf einen Wert zwischen auf 1 - 2 durch Abkühlen der Reaktionslösung auskristallisiert und abgetrennt werden.

Durch Versuche wurde gefunden, daß eine Entbenzylierung von Dibenzylbiotin durchgeführt werden kann, wenn anstelle von teurer Bromwasserstoffsäure preiswerte 70 bis 80 %ige Schwefelsäure verwendet wird. Überraschenderweise verläuft die Abspaltung der Benzylgruppen ohne daß die Carbonylgruppen angegriffen werden. Auf diese Weise kann vorteilhafterweise der Verfahrensschritt der Phosgenierung im Anschluß der Entbenzylierung entfallen. In einer recht kurzen Reaktionszeit von nur 2 bis 4 Stunden läuft die Umsetzung bei einer Temperaturen von 105 bis 125°C ab. Tränenreizende Stoffe fallen nicht an, da keine Bromierung der abgehenden Benzylgruppen erfolgen kann.

Weiterhin gestaltet sich die Aufarbeitung des D-(+)-Biotins wesentlich einfacher als nach der Entbenzylierung mit Bromwasserstoff:

Im Anschluß an die Entbenzylierung wird zu dem Reaktionsgemisch ein geeignetes organisches Lösungsmittel wie z. B. Xylol, Toluol oder zugefügt. Der pH-Wert der Lösung wird neutral bis alkalisch eingestellt. Dieses kann durch Zusatz einer Base aus der Gruppe NaOH, KOH in Form einer verdünnten wäßrigen Lösung erfolgen. Nach der Phasentrennung und einer weiteren Extraktion der organischen Phase mit einer basischen Lösung wird der pH-Wert der wäßrigen Phase auf etwa 5,5 bis 6,0 eingestellt. Anschließend wird diese Lösung mit Aktivkohle behandelt. Dann wird bei einer Temperatur von etwa 80 °C der pH-Wert mit Hilfe von Schwefelsäure langsam auf 1,3 abgesenkt und die so erhaltene Lösung langsam auf eine Temperatur von 5 °C abgesenkt, und das D-(+)-Biotin auskristallisiert.

Auf diese Weise wird D-(+)-Biotin mit einer Reinheit von 99 % der Theorie erhalten, was annähernd Lebensmittelqualität entspricht. Durch Umkristallisieren läßt sich die Reinheit noch weiter erhöhen.

Die nachfolgenden Beispiele werden zur näheren Erläuterung des erfindungsgemäßen Verfahrens gegeben.

### Beispiele

### Vergleichsbeispiel

### Herstellung von Biotin aus Dibenzylbiotin mit Bromwasserstoffsäure:

320 g rohes Dibenzylbiotin (enthält ca. 80 % reines Dibenzylbiotin 0,6 mol) wird mit 1200 g 40 %tiger Bromwasserstoffsäure versetzt und 48 h unter Rückfluß gekocht. Hierbei entstehendes Benzylbromid (ca. 200 g) wird mit Hilfe eines Wasserauskreisers als Unterphase abgetrennt. Danach wird die überschüssige Bromwasserstoffsäure abdestilliert. Der Rückstand wird mit 1 I Wasser und 300 ml Xylol aufgenommen und auf etwa 90 °C erhitzt. Danach wird mit Natronlauge ein pH-Wert von 9 eingestellt und die Phasen werden getrennt. Die wäßrige Phase wird auf die Hälfte eingeengt und mit Natronlauge ein pH-Wert von 12 eingestellt. Danach wird die Apparatur evakuiert. Unter Konstanthalten des pH-Wertes von 12 werden bei 30°C 130 g Phosgen langsam in die evakuierte Apparatur eingeblasen. Nach beendeter Reaktion wird das Vakuum gebrochen und der pH-Wert mit Salpetersäure oder mit Schwefelsäure auf 7.0 gesenkt. Nach Zusatz von 5 g Aktivkohle wird noch der Ansatz blank filtriert, auf 80 °C angeheizt und der pH-Wert mit Salpetersäure (oder Schwefelsäure) langsam auf 1,5 abgesenkt. Nach Kühlen über Nacht wird das ausgefallene Biotin abgesaugt und getrocknet. Es werden 135 g Biotin (Gehalt: ca. 93% entsprechend 125 g reinem Biotin = 0,51 mol, 85 % d. Th.) erhalten.

### Beispiel 1

### Herstellung von Biotin aus Dibenzylbiotin mit Schwefelsäure:

320 g rohes Dibenzylbiotin (enthält ca. 80 % reines Dibenzylbiotin 0,6 mol) wird mit 125 g Wasser versetzt und unter Rühren vorsichtig mit 500 g Schwefelsäure so versetzt, daß die Temperatur des Ansatzes 115 °C nicht übersteigt. Danach wird 3 Stunden bei 115 °C weitergerührt. Nach Zusatz von 2,5 I Xylol wird mit 5 I 9 %iger Natronlauge zuerst neutralisiert und danach alkalisch gestellt. Die wäßrige Phase wird abgetrennt. Die Xylol-Phase wird noch zweimal mit je 0,5 I9 %iger Natronlauge extrahiert. Die wäßrige Phase und die Natronlauge-Phasen der Nachreaktionen werden vereinigt und bei 80 °C mit Schwefelsäure auf einen pH-Wert von 6 eingestellt. Nach Zusatz von 12 g Aktivkohle wird blank filtriert und der pH-Wert mit Schwefelsäure langsam auf 1,5 abgesenkt. Nach Kühlen über Nacht wird das ausgefallene Biotin abgesaugt und getrocknet. Es werden 128 g Biotin (Gehalt: ca. 99 % entsprechend 126 g reinem Biotin = 0,52 mol, 86 % d. Th.) erhalten.

## Patentansprüche

1. Verfahren zur Herstellung von D-(+)-Biotin durch selektive Abspaltung von Benzylgruppen, indem
a) das als Zwischenprodukt gebildete Dibenzylbiotin mit Wasser versetzt und bei Temperaturen zwischen 25 bis 115°C Schwefelsäure zugefügt wird,
b) unerwünschte Neben- und Spaltprodukte nach Einstellung eines alkalischen pH-Werts mit Hilfe eines organischen Lösungsmittels extrahiert werden und
c) das freigesetzte D-(+)-Biotin durch Einstellung eines sauren pH-Werts und Erniedrigung der Temperatur auskristallisiert und abgetrennt wird.

2. Verfahren gemäß Anspruch 1, daß ungereinigtes Dibenzylbiotin verwendet wird.

3. Verfahren nach einem oder mehreren der Ansprüche 1 bis 2, **dadurch gekennzeichnet, daß** 70 bis 80 %ige Schwefelsäure verwendet wird.

4. Verfahren nach einem oder mehreren der Ansprüche 1 bis 3, **dadurch gekennzeichnet, daß** nach beendeter Reaktion unerwünschte Neben- und Spaltprodukte nach Einstellung eines alkalischen pH-Werts mit Hilfe eines organischen Lösungsmittels aus der Gruppe Toluol, Xylol extrahiert werden.

5. Verfahren nach einem oder mehreren der Ansprüche 1 bis 4, **dadurch gekennzeichnet, daß** nach erfolgter Lösungsmittelextraktion der pH-Wert schwach sauer ein gestellt wird und die wäßrige D-(+)-Biotin-haltige Lösung mit Aktivkohle behandelt wird.

6. Verfahren gemäß Anspruch 5, **dadurch gekennzeichnet, daß** der pH-Wert auf 6 eingestellt wird.

7. Verfahren nach einem oder mehreren der Ansprüche 1 bis 6, **dadurch gekennzeichnet, daß** das D-(+)-Biotin nach der Einstellung des pHs auf einen Wert zwischen 1 - 2 durch Abkühlen der Reaktionslösung auskristallisiert und abgetrennt wird.

## Claims

1. Process for the preparation of D-(+)-biotin by selective removal of benzyl groups by
a) adding water to the dibenzylbiotin formed as intermediate and adding sulfuric acid at temperatures of between 25 and 115°C.
b) extracting undesired by-products and cleavage products with the aid of an organic solvent after an alkaline pH has been established, and
c) crystallizing-out the liberated D-(+)-biotin by establishing an acidic pH and reducing the temperature, and separating the D-(+)-biotin off.

2. Process according to Claim 1, **characterized in that** impure dibenzylbiotin is used.

3. Process according to one or more of Claims 1 to 2, **characterized in that** 70 to 80% sulfuric acid is used.

4. Process according to one or more of Claims 1 to 3, **characterized in that**, when the reaction is complete, undesired by-products and cleavage products are extracted with the aid of an organic solvent from the group consisting of toluene and xylene after an alkaline pH has been established.

5. Process according to one or more of Claims 1 to 4, **characterized in that**, when the solvent extraction is complete, the pH is adjusted to weakly acidic, and the aqueous D-(+)-biotin-containing solution is treated with active carbon.

6. Process according to Claim 5, **characterized in that** the pH is adjusted to 6.

7. Process according to one or more of Claims 1 to 6, **characterized in that** the D-(+)-biotin is crystallized out by cooling the reaction solution after the pH has been adjusted to a value of 1 - 2 and is separated off.

## Revendications

1. Procédé pour la préparation de la D-(+)-biotine par séparation sélective des groupes benzyles,
a) en additionnant de l'eau à la dibenzylbiotine formée comme produit intermédiaire et en ajoutant de l'acide sulfurique à une température comprise entre 25 et 115°C,
b) en extrayant à l'aide d'un solvant organique les produits secondaires et les produits de dissociation indésirables, après ajustage d'un pH alcalin et
c) en cristallisant la D-(+)-biotine libérée par ajustage d'un pH acide et par abaissement de la température et en la séparant.

2. Procédé selon la revendication 1, **caractérisé en ce que** l'on utilise de la dibenzylbiotine non purifiée.

3. Procédé selon l'une ou plusieurs des revendications 1 à 2, **caractérisé en ce que** l'on utilise de l'acide sulfurique à 70-80 %.

4. Procédé selon l'une ou plusieurs des revendications 1 à 3, **caractérisé en ce que** l'on extrait, une fois la réaction achevée, à l'aide d'un solvant organique du groupe toluène, xylène, les produits secondaires et les produits de dissociation indésirables, après ajustage d'un pH alcalin.

5. Procédé selon l'une ou plusieurs des revendications 1 à 4, **caractérisé en ce que** l'on ajuste, après extraction avec le solvant, à un pH faiblement acide et en de que l'on traite avec du charbon actif la solution aqueuse contenant la D-(+)-biotine.

6. Procédé selon la revendication 5, **caractérisé en ce que** l'on ajuste le pH à 6.

7. Procédé selon l'une ou plusieurs des revendications 1 à 6, **caractérisé en ce que** l'on cristallise la D-(+)-biotine, après ajustage du pH à une valeur comprise entre 1 et 2, en refroidissant la solution réactionnelle et **en ce que** l'on sépare la D-(+)-biotine.
